(19) Europäisches Patentamt / European Patent Office / Office européen des brevets

(11) **EP 3 820 359 B1**

(12) **EUROPEAN PATENT SPECIFICATION**

(45) Date of publication and mention
of the grant of the patent:
**24.05.2023 Bulletin 2023/21**

(21) Application number: **19752592.6**

(22) Date of filing: **09.07.2019**

(51) International Patent Classification (IPC):
*A61B 5/021* (2006.01)    *A61B 5/0215* (2006.01)
*A61B 5/0295* (2006.01)    *A61B 7/04* (2006.01)

(52) Cooperative Patent Classification (CPC):
**A61B 5/02108; A61B 5/02158; A61B 5/0295;
A61B 7/04**

(86) International application number:
**PCT/US2019/040939**

(87) International publication number:
**WO 2020/014184 (16.01.2020 Gazette 2020/03)**

(54) **HEART SOUNDS AND PLETHYSMOGRAPHY BLOOD PRESSURE MEASUREMENT**

BLUTDRUCKMESSUNG MIT HERZTÖNEN UND PLETHYSMOGRAPHIE

MESURE DES SONS CARDIAQUES ET DE LA TENSION ARTÉRIELLE PAR PLÉTHYSMOGRAPHIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.07.2018 US 201862695511 P**

(43) Date of publication of application:
**19.05.2021 Bulletin 2021/20**

(73) Proprietor: **Cardiac Pacemakers, Inc.**
**St. Paul, Minnesota 55112-5798 (US)**

(72) Inventors:
• **STAHMANN, Jeffrey E.**
**Ramsey, Minnesota 55303 (US)**
• **THAKUR, Pramodsingh Hirasingh**
**Woodbury, Minnesota 55129 (US)**

(74) Representative: **Pfenning, Meinig & Partner mbB
Patent- und Rechtsanwälte
Joachimsthaler Straße 10-12
10719 Berlin (DE)**

(56) References cited:
WO-A1-2018/099427    DE-A1-102015 012 351
US-A- 5 054 493    US-A1- 2017 367 659
US-B1- 9 820 696

• **VIKRAM CHANDRASEKARAN ET AL: "Cuffless
Differential Blood Pressure Estimation Using
Smart Phones", IEEE TRANSACTIONS ON
BIOMEDICAL ENGINEERING, IEEE SERVICE
CENTER, PISCATAWAY, NJ, USA, vol. 60, no. 4,
1 April 2013 (2013-04-01), pages 1080-1089,
XP011497327, ISSN: 0018-9294, DOI:
10.1109/TBME.2012.2211078**

EP 3 820 359 B1

**Description**

CLAIM OF PRIORITY

This application claims the benefit of priority under 35 U.S.C. § 119(e) of U.S. Provisional Patent Application Serial Number 62/695,511, filed on July 9, 2018.

TECHNICAL FIELD

This document relates generally to medical devices, and more particularly, but not by way of limitation, to systems and devices for blood pressure measurement using heart sounds and plethysmography.

BACKGROUND

Blood pressure is the pressure of circulating blood on the walls of blood vessels, and typically refers to the pressure in large arteries of the systemic system. When further specified, such as left ventricular (LV) pressure, etc., such pressure refers to the pressure in that physiologic component. Blood pressure is commonly expressed in terms of systolic and diastolic pressure. Systolic pressure refers to the maximum pressure during a heart contraction, and diastolic pressure refers to the minimum pressure between to heart contractions, each measured in millimeters of mercury (mmHg).

An estimated 75 million patients in the United States alone suffer from hypertension, or high blood pressure. Further, such condition is poorly controlled in roughly half of such patients. High blood pressure is a risk factor for mortality, as well as other adverse medical events, including, for example, congestive heart failure, ischemia, arrhythmia, stroke, acute cardiac decompensation, organ failure, chronic kidney disease, etc. High blood pressure is also asymptomatic, so patients don't appreciate their condition until an adverse medical event occurs. Accordingly, it is important to monitor blood pressure information, such as to monitor or assess patient condition or status, including worsening or recovery of one or more physiologic conditions, diseases, patient status, or to supplement one or more other detections or determinations.

Document WO 2018/099427 A1 describes a dynamic measurement device having a function for determining a blood pressure. The device comprises: a heart beat sensing module, disposed at the position of a chest, and used for obtaining a heart beat signal by means of a heart sound sensor; a pulse sensing module, disposed at the position of a limb, and used for obtaining a pulse signal by means of a pulse wave sensor; and a data computing module, used for computing an average artery pressure, a systolic pressure and a diastolic pressure by using a heart beat signal and a pulse signal. The dynamic measurement device can dynamically monitor the blood pressure of a user in 24 hours, and can be separately used to monitor heart sounds of the user in 24 hours, so as to obtain valvular insufficiency and other heart sound anomalies, or control body condition of a hypertensive patient.

Document US 2017/367659A1 describes dynamic calibration of a blood pressure measurement device. Examples described therein are provided to enable calibrating a non-invasive blood pressure measurement device by determining multiple parameters defining a stress-strain relationship of an artery of a patient. The device may obtain output signals from a blood pressure sensor at two or more measurement elevations. The obtained measurement signals may be filtered into AC and quasi-DC components, and results fit to exponential functions to calculate an arterial time constant and a veinous time constant related to vein draining/filling rates. The arterial and veinous time constants may be used to calculate an infinity ratio. The infinity ratio and the obtained sensor output may be used to calculate values for multiple parameters defining a stress-strain relationship of a measured artery. Once defined, this stress-strain relationship may be stored and applied to future sensor output signals (e.g., blood pressure measuring sessions) to infer patient blood pressure.

SUMMARY

This document discusses, among other things, systems and methods to determine a blood pressure measurement of a subject, such as a systolic blood pressure of a subject, a diastolic blood pressure of the subject, or both, using received heart sound information and plethysmography information of the subject. The system can include a signal receiver circuit configured to receive the heart sound information and plethysmography information of the subject, and an assessment circuit configured to determine the systolic and diastolic blood pressure of the subject using the received heart sound information and the plethysmography information of the subject. The invention is defined in the appended claims.

A system in accordance with the invention includes a signal receiver circuit configured to receive heart sound information of a subject and plethysmography information of the subject; and an assessment circuit configured to determine a systolic blood pressure of the subject and to determine a diastolic blood pressure of the subject using the received heart sound information and the received plethysmography information.

The signal receiver circuit is configured to receive second heart sound (S2) information of the subject, and the assessment circuit is configured to: determine an indication of pulse pressure of the subject using the received plethysmography information; determine an indication of blood pressure of the subject using the received S2 information; and determine the systolic blood pressure of the subject and the diastolic blood pressure of the subject using the determined indication of pulse pressure of the subject and the determined indication of blood pressure of the subject.

The assessment circuit is configured to: determine a mean blood pressure of the subject using the received S2 information; and determine the systolic blood pressure of the subject and the diastolic blood pressure of the subject using the determined indication of pulse pressure of the subject and the determined mean blood pressure of the subject.

The assessment circuit may be configured to determine the systolic blood pressure as a function of the determined mean blood pressure of the subject and the determined indication of pulse pressure of the subject, and to determine the diastolic blood pressure as a further function of the determined mean blood pressure of the subject and the determined indication of pulse pressure of the subject, wherein the function is different than the further function.

The assessment circuit is configured to determine the systolic blood pressure as an increase to the mean blood pressure by a first function of the determined indication of pulse pressure of the subject, and to determine the diastolic blood pressure as a decrease from the mean blood pressure by a second function of the determined indication of pulse pressure of the subject.

The assessment circuit is configured to determine the first function as a function of a rise time of the plethysmography signal and a time between the S2 heart sound and a peak time of the plethysmography signal.

In an example, the assessment circuit may optionally be configured to determine the first and second functions as different functions of a rise time of the plethysmography signal and a time between the S2 heart sound and a time at or near the peak of the plethysmography signal.

In an example, the second heart sound (S2) information may include at least one of a second heart sound (S2) amplitude, energy, or time.

In an example, the system may optionally be configured to include: a heart sound sensor configured to detect heart sound information from the subject and to determine second heart sound (S2) information using the detected heart sound information; and a plethysmography sensor configured to detect plethysmography information from the subject, wherein the signal receiver circuit is configured to receive the determined second heart sound (S2) information from the heart sound sensor, and to receive the detected plethysmography information from the plethysmography sensor.

At least one machine-readable medium in accordance with the invention includes instructions that, when performed by a medical device, cause the medical device to perform operations comprising: receiving heart sound information of a subject and plethysmography information of the subject; and determining a systolic blood pressure of the subject and a diastolic blood pressure of the subject using the received heart sound information and the received plethysmography information.

The at least one machine-readable medium is configured such that receiving heart sound information comprises receiving second heart sound (S2) information of the subject, and the instructions, when performed by the medical device, cause the medical device to perform operations comprising: determining an indication of pulse pressure of the subject using the received plethysmography information; determining an indication of blood pressure of the subject using the received S2 information, and determining the systolic blood pressure and the diastolic blood pressure comprises determining the systolic blood pressure of the subject and the diastolic blood pressure of the subject using the determined indication of pulse pressure of the subject and the determined indication of blood pressure of the subject.

The at least one machine-readable medium is configured such that the instructions, when performed by the medical device, cause the medical device to perform operations comprising: determining a mean blood pressure of the subject using the received S2 information; and wherein determining the systolic blood pressure and the diastolic blood pressure comprises determining the systolic blood pressure of the subject and the diastolic blood pressure of the subject using the determined indication of pulse pressure of the subject and the determined mean blood pressure of the subject.

The at least one machine-readable medium may be configured such that determining the systolic blood pressure comprises determining the systolic blood pressure of the subject as a function of the determined mean blood pressure of the subject and the determined indication of pulse pressure of the subject, determining the diastolic blood pressure comprises determining the diastolic blood pressure of the subject as a further function of the determined mean blood pressure of the subject and the determined indication of pulse pressure of the subject, and the function is different than the further function.

The at least one machine-readable medium is configured such that determining the systolic blood pressure comprises determining the systolic blood pressure of the subject as an increase to the mean blood pressure by a first function of the determined indication of pulse pressure of the subject, and determining the diastolic blood pressure comprises determining the diastolic blood pressure of the subject as a decrease from the mean blood pressure by a second function of the determined indication of pulse pressure of the subject.

The at least one machine-readable medium may be configured such that the instructions, when performed by the medical device, cause the medical device to perform operations comprising: determining the first and second functions as different functions of a rise time of the plethysmography signal and a time between the S2 heart sound and a time at or near the peak of the plethysmography signal.

An non-claimed example of a method may include: receiving heart sound information of a subject and plethysmography information of the subject using a signal receiver circuit; and determining, an assessment circuit, a systolic blood pressure of the subject and a diastolic blood pressure of the subject using the received heart sound information and the received plethysmography information.

The non-claimed method may optionally be configured such that receiving heart sound information includes receiving second heart sound (S2) information of the subject, wherein the method comprises: determining an indication of pulse pressure of the subject using the received plethysmography information; determining an indication of blood pressure of the subject using the received S2 information; and determining the systolic blood pressure and the diastolic blood pressure comprises determining the systolic blood pressure of the subject and the diastolic blood pressure of the subject using the determined indication of pulse pressure of the subject and the determined indication of blood pressure of the subject.

The non-claimed method may optionally be configured such that determining a mean blood pressure of the subject using the received S2 information; and determining the systolic blood pressure of the subject and the diastolic blood pressure of the subject using the determined indication of pulse pressure of the subject and the determined mean blood pressure of the subject.

The non-claimed method may optionally be configured such that determining the systolic blood pressure includes determining the systolic blood pressure of the subject as a first function of the determined mean blood pressure of the subject and the determined indication of pulse pressure of the subject, and determining the diastolic blood pressure includes determining the diastolic blood pressure of the subject as a second function of the determined mean blood pressure of the subject and the determined indication of pulse pressure of the subject, and wherein the first function is different than the second function.

The non-claimed method may optionally be configured such that determining the systolic blood pressure includes determining the systolic blood pressure of the subject as an increase to the mean blood pressure by a first function of the determined indication of pulse pressure of the subject, and determining the diastolic blood pressure includes determining the diastolic blood pressure of the subject as a decrease from the mean blood pressure by a second function of the determined indication of pulse pressure of the subject.

The non-claimed method may optionally be configured to include determining the first and second functions as different functions of a rise time of the plethysmography signal and a time between the S2 heart sound and a time at or near the peak of the plethysmography signal.

An example of subject matter (e.g., a system or apparatus may optionally combine any portion or combination of any portion of any one or more of the aforementioned system, at least one machine-readable medium or non-claimed method to include "means for" performing any portion of any one or more of the functions or methods of the aforementioned system, at least one machine-readable medium or non-claimed method, or a "non-transitory machine-readable medium" including instructions that, when performed by a machine, cause the machine to perform any portion of any one or more of the functions or methods of the aforementioned system, at least one machine-readable medium or non-claimed method.

This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

FIG. 1 illustrates an example relationship between physiologic signals of a subject.
FIG. 2 illustrates an example system including an ambulatory medical device (AMD) configured to sense or detect information from a subject.
FIG. 3 illustrates an example system including a signal receiver circuit and an assessment circuit.
FIGS. 4-5 illustrate example systems including an ambulatory medical device (AMD) and an external system.
FIGS. 6-7 illustrate non-claimed example methods including determining a systolic blood pressure and a diastolic blood pressure.

FIG. 8 illustrates a block diagram of an example machine upon which any one or more of the techniques discussed herein may perform.

DETAILED DESCRIPTION

Traditional blood pressure measurements include those taken non-invasively, such as using a mercury manometer, or a blood pressure cuff. However, such measurements can be burdensome, are often inaccurate, discontinuous (e.g., hourly or daily intervals, etc.), and when used for ambulatory or chronic measurements, often suffer from lack of patient compliance. In contrast, implanted systems including a blood pressure sensor that continuously measures blood pressure can be unnecessarily invasive or require additional, otherwise unnecessary sensors, increasing system complexity and cost. For example, an implanted pressure sensor is not appropriate for outpatient or long-term use. Moreover, implanted systems that continuously determine or infer blood pressure (e.g., at intervals more frequent than hourly or daily, such as at each cardiac cycle, or periods of cardiac cycles, etc.) using one or more other sensors can be inaccurate, in certain examples, including periods of noise or inaccurate measurement, or require frequent and costly calibration to maintain accuracy. It can be beneficial to more accurately determine blood pressure, in certain examples, reducing such periods of noise or inaccurate measurement, and moreover, to determine blood pressure using existing, dual-purpose, or multi-use sensors, in certain examples, different from a dedicated pressure sensor, reducing cost or complexity of ambulatory systems. Further, it can be beneficial to continuously determine blood pressure, at each cardiac cycle, or at each qualifying cardiac cycle.

Heart sounds are recurring mechanical signals associated with cardiac vibrations from blood flow through the heart with each cardiac cycle and can be separated and classified according to activity associated with the vibrations and blood flow. Heart sounds include four major sounds: the first through the fourth heart sounds. The first heart sound (S 1) is the vibrational sound made by the heart during closure of the atrioventricular (AV) valves, the mitral valve and the tricuspid valve, at the beginning of systole. The second heart sound (S2) is the vibrational sound made by the heart during closure of the aortic and pulmonary valves at the beginning of diastole. The third and fourth heart sounds (S3, S4) are related to filling pressures of the left ventricle during diastole.

Filling of the left ventricle from the left atrium begins as the left ventricle relaxes following a contraction, and the pressure in the left ventricle falls below the pressure of the left atrium, opening the mitral valve. As the left ventricle contracts, the pressure in the left ventricle quickly rises. When the pressure in the left ventricle rises above the pressure of the left atrium, the mitral valve snaps shut, isolating the left ventricle from the left atrium, resulting in the first heart sound. Close in time to the closure of the mitral valve, when the pressure in the left ventricle rises above the pressure of the aorta, the aortic valve opens, allowing blood to exit the left ventricle for the rest of the body through the aorta. The maximum pressure in the left ventricle during contraction, the systolic pressure, is representative of the maximum systemic pressure in the arteries following contraction of the left ventricle (e.g., typically 100 - 140 mmHg, etc.).

As the left ventricle relaxes, the pressure in the left ventricle drops. When the pressure in the left ventricle falls below the pressure of the aorta, the aortic valve snaps shut, isolating the left ventricle from the aorta, resulting in the second heart sound. The pressure in the arterial system at the time of the aortic valve opening is the systemic diastolic pressure (e.g., typically 60 - 100 mmHg, etc.). Close in time to the closure of the aortic valve, when the pressure in the left ventricle falls below the pressure of the left atrium, the mitral valve opens, filling the left ventricle. The minimum pressure in the left ventricle following contraction is the left ventricular diastolic pressure (e.g., typically down to 5 - 10 mmHg, etc.), different in amplitude, and possibly time, than the systemic diastolic pressure.

The heart valves change states between open and closed at various times during the cardiac cycle. These valve state changes occur when specific relative pressures are present within the heart and the major vessels leading from the heart (e.g., the aorta). Both the valve state changes and the effects of the state changes are detectable through various methods. For example, valve closure cause vibrations of the walls of the heart that can be detected using an accelerometer or a microphone. The movement of the valves can be detected directly via imaging technologies such as echocardiography and magnetic resonance imaging (MRI) or by intracardiac impedance plethysmography.

Various physiologic conditions can be detected using heart sounds, including, for example, acute physiologic events, such as one or more abnormal cardiac rhythms (e.g., atrial fibrillation, atrial flutter, cardiac mechanical dyssynchrony, etc.), as well as more chronic physiologic events, such as congestive heart failure, ischemia, etc.

Further, heart sounds can be correlated with certain physiologic information, such that, in certain examples, heart sound information can be used as a surrogate for, or to detect one or more physiologic characteristics. For example, heart sounds can be used to detect atrial filling pressure, such as illustrated in the commonly assigned Siejko et al. U.S. Patent No. 7,972,275, titled "Method and Apparatus for Monitoring of Diastolic Hemodynamics", or the commonly assigned Patangay et al. U.S. Patent No. 8,048,001, titled "Method and Apparatus for Detecting Atrial Filling Pressure".

Heart sounds are generally related to blood pressure. Chronic monitoring of blood pressure based on the frequency and/or amplitude components of first and second heart sounds have been proposed. However, the present inventors have recognized, among other things, that the relationship between heart sounds and blood pressure changes according to different, interdependent variables, that in certain examples, heart sounds track blood pressure, but other times they do not, and that, accordingly, certain ventricular functions or physiologic information can be used to identify periods of increased and decreased correlation between blood pressure and heart sounds. The increase and decrease in correlation can be used to increase the sensitivity or specificity of blood pressure detection using heart sounds, or to increase the efficiency of data collection and storage to accurately monitor blood pressure using heart sounds. Accordingly, the non-claimed methods and the systems described herein can provide a for more robust blood pressure monitoring, in certain examples, using less storage or data processing than existing ambulatory systems or devices.

The present inventors have recognized, among other things, that S2 measurements are more correlative to aortic blood pressure across subjects during certain conditions. For example, a raw S2 signal (e.g., amplitude) has a first correlation (e.g., coefficient of determination ($R^2$)) to aortic pressure that varies across subjects and within specific subjects over time (e.g., $R^2$ = 0.09 - 0.74). The correlation increases with different measurements of S2. For example, filtering noise, such as using a median filter (e.g., 10-point median filter, short-term (~5min) median filter, etc.), increases correlation (e.g., $R^2$ = 0.56 - 0.76). In other examples, one or more other heart sounds can be used to determine which S2 measurements to use to determine blood pressure measurements. For example, limiting S2 measurements to periods (cardiac cycles) of S 1 increase less than a threshold (e.g., less than 25% of a median S1 measurement (e.g., amplitude), etc.) can further increase correlation of S2 to aortic pressure (e.g., $R^2$ = 0.74 - 0.84). In certain examples, though, heart sounds provide a measurement of one of mean blood pressure, diastolic blood pressure, or systolic blood pressure, but not the others.

Plethysmography is the measurement of a change in volume in a body, typically air or fluid (e.g., blood). Changes in blood volume can provide a relative measure of pulse pressure, the difference between systolic and diastolic blood pressure, of a subject. Two methods of measuring blood volume plethysmography of a subject are photo plethysmography, measuring changes in blood volume optically using a light sensor (and typically a light source), or impedance plethysmography, measuring changes in blood volume electrically using one or more electrodes. Further, changes in plethysmography measurements have a high correlation to changes in pulse pressure (e.g., $R^2$ = 0.7121). The correlation between plethysmography measurements and pulse pressure increases during periods of arrhythmia (e.g., $R^2$ = 0.7858).

In certain examples, heart sounds can provide a measurement of one of mean blood pressure, diastolic blood pressure, or systolic blood pressure, but not the others. The present inventors have recognized, among other things, that indications of pulse pressure, such as plethysmograph information, can be used to determine diastolic and systolic blood pressure using an indication of mean blood pressure, to determine mean blood pressure and diastolic blood pressure using an indication of systolic blood pressure, or to determine mean blood pressure and systolic blood pressure using an indication of diastolic blood pressure.

For example:

$$Pulse\ Pressure\ (PP) = Systolic\ Blood\ Pressure\ (BP) - Diastolic\ BP$$

$$(1)$$

$$Mean\ BP = (Systolic\ BP + 2(Diastolic\ BP))/3$$

$$(2)$$

If mean blood pressure can be detected using heart sound information (e.g., S2 at specific conditions of S1, etc.), and pulse pressure can be detected using plethysmography information, the two unknown quantities in equations (1) and (2) can be solved as:

$$Systolic\ BP = Mean\ BP + (2(PP)/3)$$

$$(3)$$

$$Diastolic\ BP = Mean\ BP - (PP/3)$$

$$(4)$$

Similar equations can be solved if one or more of systolic or diastolic blood pressure are known.

In certain examples, systolic blood pressure or diastolic blood pressure can be determined by linear (or non-linear) extrapolation with one or more other events, indicators, or physiologic information.

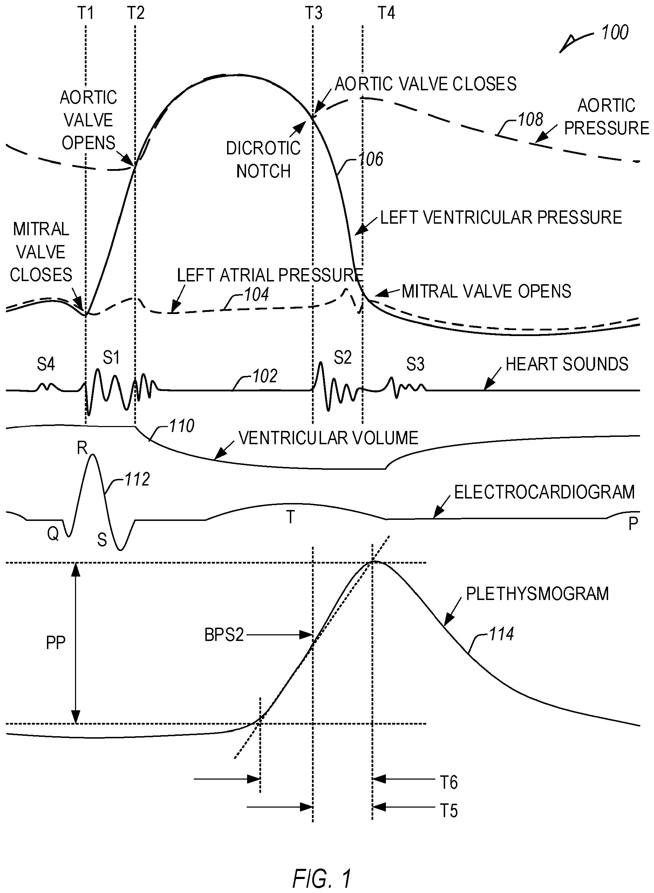

FIG. 1 illustrates an example relationship 100 between physiologic information, including heart sounds 102 (first, second, third, and fourth heart sounds (S1, S2, S3, and S4)), left atrial pressure 104, left ventricular pressure 106, aortic pressure 108, ventricular volume 110, an electrocardiogram 112, and a plethysmogram 114.

At a first time (T1), a mitral valve closes, marking a rise in left ventricular pressure 106, and the start of the first heart sound (S1) and systole, or ventricular contraction. At a second time (T2), an aortic valve opens, marking a rise in aortic pressure 108, a drop in ventricular volume 110, and continuing S1. S1 is caused by closure of the atrioventricular (AV) valves, including the mitral and tricuspid valves, and can be used to monitor heart contractility. As the left ventricular pressure 106 falls, the plethysmogram 114 rises.

At a third time (T3), an aortic valve closes, causing a dicrotic notch in the aortic pressure 108 and the second heart sound (S2), and marking the end of systole, or ventricular contraction, and the beginning of diastole, or ventricular relaxation. S2 can be used to monitor blood pressure. At a fourth time (T4), the mitral valve opens, the left atrial pressure 104 drops, and the ventricular volume 110 increases. An abrupt halt of early diastolic filling can cause the third heart sound (S3), which can be indicative of (or an early sign of) heart failure (HF). As the left ventricular pressure 106 relaxes, and the ventricular volume 110 increases, the plethysmogram 114 falls. Vibrations due to atrial kick can cause the fourth heart sound (S4), which can be used to monitor ventricular compliance.

Systolic time intervals, such as pre-ejection period (PEP) or left ventricular ejection time (LVET) can be indicative of clinically relevant information, including contractility, arrhythmia, Q-T prolongation (with electrogram (EGM) information), etc. The PEP can be measured from a Q wave of an EGM to the time of the aortic valve opening, T2 in FIG. 1. The LVET can include a time between the aortic valve opening, T2, and the aortic valve closing, T3. In other examples, one or more systolic time intervals can be detected and used to detect physiologic information of a subject (e.g., PEP/LVET, one or more mechanical, electrical, or mechanical-electrical time intervals, etc.).

The PP of the subject can be determined using the plethysmogram 114. The change in the plethysmogram 114 can be correlative to a change in pulse pressure (PP) of the subject, in certain examples, measured as the difference between a peak and a trough of the plethysmogram 114. A relative measure of PP, or mean PP, can be determined proportionate to the change in the plethysmogram 114, or a value in $\Delta$mmHg can be determined as a relative measure of the PP, by calibrating the PP to the plethysmogram 114 (e.g., $\Delta$PP), or using population-based measurements or measurements from one or more subjects. T5 can be a time between S2 (or T3) and a peak of the plethysmogram 114. The BP at S2 ($BP_{S2}$) can be determined using a measure of S2, or the plethysmogram 114 at S2 (or T3). T6 can include a rising time of the plethysmogram 114.

$$Systolic\ BP = BP_{S2} + a(PP)$$

$$(5)$$

$$Diastolic\ BP = BP_{S2} - (1\text{-}a)PP$$

$$(6)$$

$$a = (T6\text{-}T5)/T6$$

$$(7)$$

In certain examples, the relationship between systolic blood pressure and diastolic blood pressure can be linear. In other examples, the relationship between systolic blood pressure and diastolic blood pressure can be non-linear (e.g., sigmoid, etc.). The functions used to determine the systolic blood pressure and diastolic blood pressure (e.g., *a*, from equation (7)) can be linear or non-linear, etc. In other examples, one or more other functions can be used to determine the systolic and diastolic blood pressure.

Ambulatory medical devices, including implantable or wearable medical devices configured to monitor, detect, or treat various cardiac conditions associated with a reduced ability of a heart to sufficiently deliver blood to a body, such as heart failure (HF), arrhythmias, hypertension, etc. Heart sound sensors and plethysmography sensors can be components of the same or different ambulatory medical devices (AMDs). Various ambulatory medical devices can be implanted in a subject's body or otherwise positioned on or about the subject to monitor subject physiologic information, such as heart sounds, respiration (e.g., respiration rate, tidal volume, etc.), impedance (e.g., thoracic impedance, cardiac impedance, etc.), pressure (e.g., blood pressure), cardiac activity (e.g., heart rate), physical activity, posture, plethysmography, or one or more other physiologic parameters of a subject, or to provide electrical stimulation or one or more other therapies or treatments to optimize or control contractions of the heart.

Traditional cardiac rhythm management (CRM) devices, such as pacemakers, defibrillators, or cardiac resynchronizers, include subcutaneous devices configured to be implanted in a chest of a subject, having one or more leads to position one or more electrodes or other sensors at various locations in or near the heart, such as in one or more of the atria or ventricles. Separate from, or in addition to, the one or more electrodes or other sensors of the leads, the CRM device can include one or more electrodes or other sensors (e.g., a pressure sensor, an accelerometer, a gyroscope, a microphone, etc.) powered by a power source in the CRM device. The one or more electrodes or other sensors of the leads, the CRM device, or a combination thereof, can be configured detect physiologic information from, or provide one or more therapies or stimulation to, the subject.

In addition, implantable devices can include leadless cardiac pacemakers (LCP), including small (e.g., smaller than traditional implantable CRM devices, in certain examples having a volume of about 1 cc, etc.), self-contained devices including one or more sensors, circuits, or electrodes configured to monitor physiologic information (e.g., heart rate, etc.) from, detect physiologic conditions (e.g., tachycardia) associated with, or provide one or more therapies or stimulation to the heart without traditional lead or implantable CRM device complications (e.g., required incision and pocket, complications associated with lead placement, breakage, or migration, etc.). In certain examples, an LCP can have more limited power and processing capabilities than a traditional CRM device; however, multiple LCP devices can be implanted in or about the heart to detect physiologic information from, or provide one or more therapies or stimulation to, one or more chambers of the heart. The multiple LCP devices can communicate between themselves, or one or more other implanted or external devices.

Wearable or external medical sensors or devices can be configured to detect or monitor physiologic information of the subject without required implant or an in-patient procedure for placement, battery replacement, or repair. However, such sensors and devices, in contrast to implantable medical devices, may have reduced patient compliance, increased detection noise, or reduced detection sensitivity.

For each ambulatory medical device (AMD) described above (e.g., implantable medical device (IMD) or wearable medical devices (WMD)), each additional sensor can increase system cost and complexity, reduce system reliability, or increase the power consumption and reduce the usable life of the ambulatory device. Accordingly, it can be beneficial to use a single sensor to determine multiple types of physiologic information, or a smaller number of sensors to measure a larger number of different types of physiologic information.

In an example, an accelerometer, acoustic sensor, or other heart sound sensor can be used to determine heart sound information of the subject, as well as blood pressure information or one or more other types of physiologic information of the subject. A plethysmography sensor (e.g., a photoplethysmography (PPG) sensor, an impedance plethysmography sensor, etc.) can be used to determine information indicative of a volume change of the subject, such as a change in blood volume, in certain examples indicative of pulse pressure, etc. An assessment circuit can determine blood pressure information of the subject using heart sound information, plethysmography information, or a combination of heart sound and plethysmography information, and in certain examples, determine a subject status or risk or stratification of worsening subject condition using the determined blood pressure information. The assessment circuit can provide an alert or indication to the subject or a clinician that the subject seek medical treatment or be hospitalized in response to such determination, or otherwise determine one or more therapy parameters, such as to be provided to a clinician for consideration, or to propose, control, or otherwise manage one or more therapies to the subject.

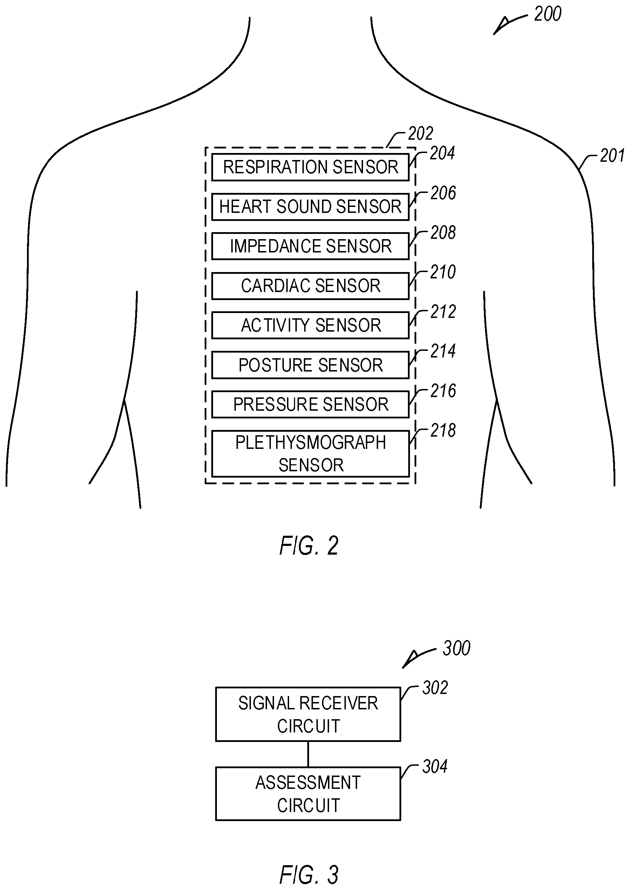

FIG. 2 illustrates an example system 200 including an ambulatory medical device (AMD) 202 configured to sense or detect information from a subject 201 (e.g., a patient). In an example, the AMD 202 can include an implantable medical device (IMD), a wearable or external medical device, or one or more other implantable or external medical devices or patient monitors. The AMD 202 can include a single device, or a plurality of medical devices or monitors configured to detect subject information.

The AMD 202 can include one or more sensors configured to receive physiologic information of a subject 201. In an example, the AMD 202 can include one or more of a respiration sensor 204 configured to receive respiration information (e.g., a respiration rate (RR), a respiration volume (tidal volume), etc.), a heart sound sensor 206 configured to receive heart sound information, an impedance sensor 208 (e.g., intrathoracic impedance sensor, transthoracic impedance sensor, etc.) configured to receive impedance information, a cardiac sensor 210 configured to receive cardiac electrical information, an activity sensor 212 configured to receive information about a physical motion (e.g., activity, steps, etc.), a posture sensor 214 configured to receive posture or position information, a pressure sensor 216 configured to receive pressure information, a plethysmograph sensor 218 (e.g., a photoplethysmography sensor, etc.), or one or more other sensors configured to receive physiologic information of the subject 201.

FIG. 3 illustrates an example system (e.g., a medical device, etc.) 300 including a signal receiver circuit 302 and an assessment circuit 304. The signal receiver circuit 302 can be configured to receive subject information, such as physiologic information of a subject, a patient (or a group of subjects or patients) from one or more sensors (e.g., such as those illustrated in FIG. 2, etc.). The assessment circuit 304 can be configured to receive information from the signal receiver circuit 302, and to determine one or more parameters (e.g., composite physiologic parameters, stratifiers, one or more pacing parameters, etc.), such as described herein.

The assessment circuit 304 can be configured to provide an output to a user, such as to a display or one or more other user interface, the output including a score, a trend, or other indication. In other examples, the assessment circuit 304 can be configured to provide an output to another circuit, machine, or process, such as to control, adjust, or cease a therapy of a medical device, a drug delivery system, etc.

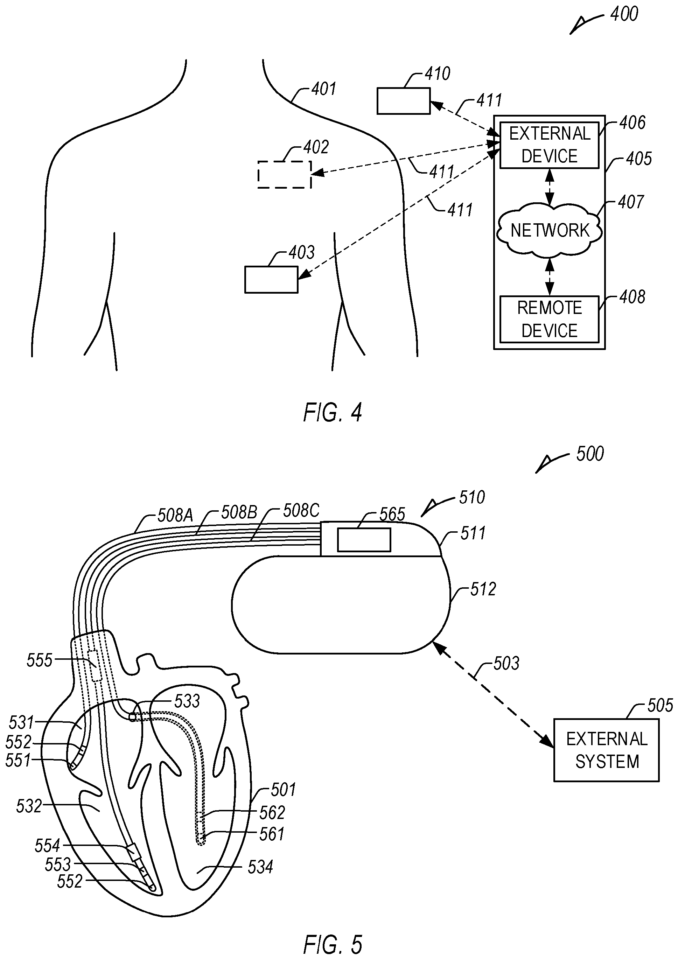

FIG. 4 illustrates an example patient management system 400 and portions of an environment in which the system 400 may operate. The patient management system 400 can perform a range of activities, including remote patient monitoring and diagnosis of a disease condition. Such activities can be performed proximal to a patient 401, such as in a patient home or office, through a centralized server, such as in a hospital, clinic, or physician office, or through a remote workstation, such as a secure wireless mobile computing device.

The patient management system 400 can include one or more ambulatory devices, an external system 405, and a communication link 411 providing for communication between the one or more ambulatory devices and the external system 405. The one or more ambulatory devices can include an implantable medical device (IMD) 402, a wearable medical device 403, or one or more other implantable, leadless, subcutaneous, external, wearable, or ambulatory medical devices configured to monitor, sense, or detect information from, determine physiologic information about, or provide one or more therapies to treat various cardiac conditions of the patient 401, such as high blood pressure, an ability of a heart to sufficiently deliver blood to a body, including atrial fibrillation (AF), congestive heart failure (CHF), hypertension, or one or more other cardiac or non-cardiac conditions (e.g., dehydration, hemorrhage, renal dysfunction, etc.).

In an example, the IMD 402 can include one or more traditional cardiac rhythm management (CRM) devices, such as a pacemaker or defibrillator, implanted in a chest of a patient, having a lead system including one or more transvenous, subcutaneous, or non-invasive leads or catheters to position one or more electrodes or other sensors (e.g., a heart sound sensor) in, on, or about a heart or one or more other position in a thorax, abdomen, or neck of the patient 401. In another example, the IMD 402 can include a monitor implanted, for example, subcutaneously in the chest of patient 401.

The IMD 402 can include an assessment circuit configured to detect or determine specific physiologic information of the patient 401, or to determine one or more conditions or provide information or an alert to a user, such as the patient 401, a clinician, or one or more other caregivers. The IMD 402 can alternatively or additionally be configured as a therapeutic device configured to treat one or more medical conditions of the patient 401. The therapy can be delivered to the patient 401 via the lead system and associated electrodes or using one or more other delivery mechanisms. The therapy can include anti-arrhythmic therapy to treat an arrhythmia or to treat or control one or more complications from arrhythmias, such as syncope, congestive heart failure (CHF), or stroke, among others. In other examples, the therapy can include delivery of one or more drugs to the patient 401 using the IMD 402 or one or more of the other ambulatory devices. Examples of the anti-arrhythmic therapy include pacing, cardioversion, defibrillation, neuromodulation, drug therapies, or biological therapies, among other types of therapies. In other examples, therapies can include cardiac resynchronization therapy (CRT) for rectifying dyssynchrony and improving cardiac function in CHF patients. In some examples, the IMD 402 can include a drug delivery system, such as a drug infusion pump to deliver drugs to the patient for managing arrhythmias or complications from arrhythmias, hypertension, or one or more other physiologic conditions. In yet other examples, the IMD 402 can include a therapy circuit or module configured to treat hypertension (e.g., a neuro-stimulation therapy circuit, a drug delivery therapy circuit, a stimulation therapy circuit, etc.).

The wearable medical device 403 can include one or more wearable or external medical sensors or devices (e.g., automatic external defibrillators (AEDs), Holter monitors, patch-based devices, smart watches, smart accessories, wrist- or finger-worn medical devices, such as a finger-based photoplethysmography sensor, etc.). The wearable medical device 403 can include an optical sensor configured to detect a photoplethysmogram (PPG) signal on a wrist, finger, or other location on the patient. In other examples, the wearable medical device 403 can include an acoustic sensor or accelerometer to detect acoustic information (e.g., heart sounds) or the sound or vibration of blood flow, an impedance sensor to detect impedance variations associated with changes in blood flow or volume, a temperature sensor to detect temperature variation associated with blood flow, a laser Doppler vibrometer or other pressure, strain, or physical sensor to detect physical variations associated with blood flow, etc.

The patient management system 400 can include, among other things, a respiration sensor configured to receive respiration information (e.g., a respiration rate (RR), a respiration volume (tidal volume), etc.), a heart sound sensor configured to receive heart sound information, a thoracic impedance sensor configured to receive impedance information, a cardiac sensor configured to receive cardiac electrical information, an activity sensor configured to receive information about a physical motion (e.g., activity, posture, etc.), a plethysmography sensor, or one or more other sensors configured to receive physiologic information of the patient 401.

The external system 405 can include a dedicated hardware/software system, such as a programmer, a remote server-based patient management system, or alternatively a system defined predominantly by software running on a standard personal computer. The external system 405 can manage the patient 401 through the IMD 402 or one or more other ambulatory devices connected to the external system 405 via a communication link 411. In other examples, the IMD 402 can be connected to the wearable device 403, or the wearable device 403 can be connected to the external system 405, via the communication link 411. This can include, for example, programming the IMD 402 to perform one or more of acquiring physiological data, performing at least one self-diagnostic test (such as for a device operational status), analyzing the physiological data to detect a cardiac arrhythmia, or optionally delivering or adjusting a therapy to the patient 401. Additionally, the external system 405 can send information to, or receive information from, the IMD 402 or the wearable device 403 via the communication link 411. Examples of the information can include real-time or stored physiological data from the patient 401, diagnostic data, such as detection of cardiac arrhythmias or events of worsening heart failure, responses to therapies delivered to the patient 401, or device operational status of the IMD 402 or the wearable device 403 (e.g., battery status, lead impedance, etc.). The communication link 411 can be an inductive telemetry link, a capacitive telemetry link, or a radio-frequency (RF) telemetry link, or wireless telemetry based on, for example, "strong" Bluetooth or IEEE 802.11 wireless fidelity "Wi-Fi" interfacing standards. Other configurations and combinations of patient data source interfacing are possible.

By way of example and not limitation, the external system 405 can include an external device 406 in proximity of the one or more ambulatory devices, and a remote device 408 in a location relatively distant from the one or more ambulatory devices, in communication with the external device 406 via a communication network 407. Examples of the external device 406 can include a medical device programmer.

The remote device 408 can be configured to evaluate collected patient information and provide alert notifications, among other possible functions. In an example, the remote device 408 can include a centralized server acting as a central hub for collected patient data storage and analysis. The server can be configured as a uni-, multi-, or distributed computing and processing system. The remote device 408 can receive patient data from multiple patients including, for example, the patient 401. The patient data can be collected by the one or more ambulatory devices, among other data acquisition sensors or devices associated with the patient 401. The server can include a memory device to store the patient data in a patient database. The server can include an alert analyzer circuit to evaluate the collected patient data to determine if specific alert condition is satisfied. Satisfaction of the alert condition may trigger a generation of alert notifications. In some examples, the alert conditions may alternatively or additionally be evaluated by the one or more ambulatory devices, such as the IMD 402. By way of example, alert notifications can include a Web page update, phone or pager call, E-mail, SMS, text or "Instant" message, as well as a message to the patient and a simultaneous direct notification to emergency services and to the clinician. Other alert notifications are possible. The server can include an alert prioritizer circuit configured to prioritize the alert notifications. For example, an alert of a detected medical event can be prioritized using a similarity metric between the physiological data associated with the detected medical event to physiological data associated with the historical alerts.

The remote device 408 may additionally include one or more locally configured clients or remote clients securely connected over the communication network 407 to the server. Examples of the clients can include personal desktops, notebook computers, mobile devices, or other computing devices. System users, such as clinicians or other qualified medical specialists, may use the clients to securely access stored patient data assembled in the database in the server, and to select and prioritize patients and alerts for health care provisioning. In addition to generating alert notifications, the remote device 408, including the server and the interconnected clients, may also execute a follow-up scheme by sending follow-up requests to the one or more ambulatory devices, or by sending a message or other communication to the patient 401, clinician or authorized third party as a compliance notification.

The communication network 407 can provide wired or wireless interconnectivity. In an example, the communication network 407 can be based on the Transmission Control Protocol/Internet Protocol (TCP/IP) network communication specification, although other types or combinations of networking implementations are possible. Similarly, other network topologies and arrangements are possible.

One or more of the external device 406 or the remote device 408 can output the detected medical events to a system user, such as the patient or a clinician, or to a process including, for example, an instance of a computer program executable in a microprocessor. In an example, the process can include an automated generation of recommendations for anti-arrhythmic therapy, or a recommendation for further diagnostic test or treatment. In an example, the external device 406 or the remote device 408 can include a respective display unit for displaying the physiological or functional signals, or alerts, alarms, emergency calls, or other forms of warnings to signal the detection of arrhythmias. In some examples, the external system 405 can include an external data processor configured to analyze the physiological or functional signals received by the one or more ambulatory devices, and to confirm or reject the detection of arrhythmias. Computationally intensive algorithms, such as machine-learning algorithms, can be implemented in the external data processor to process the data retrospectively to detect cardia arrhythmias.

Portions of the one or more ambulatory devices or the external system 405 can be implemented using hardware, software, firmware, or combinations thereof. Portions of the one or more ambulatory devices or the external system 405 can be implemented using an application-specific circuit that can be constructed or configured to perform one or more functions or can be implemented using a general-purpose circuit that can be programmed or otherwise configured to perform one or more functions. Such a general-purpose circuit can include a microprocessor or a portion thereof, a microcontroller or a portion thereof, or a programmable logic circuit, a memory circuit, a network interface, and various components for interconnecting these components. For example, a "comparator" can include, among other things, an electronic circuit comparator that can be constructed to perform the specific function of a comparison between two signals or the comparator can be implemented as a portion of a general-purpose circuit that can be driven by a code instructing a portion of the general-purpose circuit to perform a comparison between the two signals.

The patient management system 400 can include a therapy device 410, such as a drug delivery device 406 configured to provide therapy or therapy information (e.g., dosage information, etc.) to the patient 401, such as using information from one or more of the ambulatory devices. In other examples, one or more of the ambulatory devices can be configured to provide therapy or therapy information to the patient 401. The therapy device 410 can be configured to send information to or receive information from one or more of the ambulatory devices or the external system 405 using the communication link 411. In an example, the one or more ambulatory devices, the external device 406, or the remote device 408 can be configured to control one or more parameters of the therapy device 410.

FIG. 5 illustrates an example of a Cardiac Rhythm Management (CRM) system 500 and portions of an environment in which the CRM system 500 can operate. The CRM system 500 can include an ambulatory medical device, such as an implantable medical device (IMD) 510 that can be electrically coupled to a heart 501 such as through one or more leads 508A - C coupled to the IMD 510 using a header 511, and an external system 505 that can communicate with the IMD 510 such as via a communication link 503.

The IMD 510 can include an implantable cardiac device such as a pacemaker, an implantable cardioverter-defibrillator (ICD), or a cardiac resynchronization therapy defibrillator (CRT-D). The IMD 510 can include one or more monitoring or therapeutic devices such as a subcutaneously implanted device, a wearable external device, a neural stimulator, a drug delivery device, a biological therapy device, or one or more other ambulatory medical devices. The IMD 510 may be coupled to or substituted by a monitoring medical device, such as a bedside or other external monitor.

The IMD 510 can include a hermetically sealed can 512 that can house an electronic circuit that can sense a physiologic signal in the heart 501 and can deliver one or more therapeutic electrical pulses to a target region, such as in the heart, such as through one or more leads 508A - C. In certain examples, the CRM system 500 can include only a single lead, such as 508B, or can include only two leads, such as 508A and 508B.

The lead 508A can include a proximal end that can be configured to be connected to IMD 510 and a distal end that can be configured to be placed at a target location such as in the right atrium (RA) 531 of the heart 501. The lead 508A can have a first pacing-sensing electrode 551 that can be located at or near its distal end, and a second pacing-sensing electrode 552 that can be located at or near the electrode 551. The electrodes 551 and 552 can be electrically connected to the IMD 510 such as via separate conductors in the lead 508A, such as to allow for sensing of the right atrial activity and optional delivery of atrial pacing pulses. The lead 508B can be a defibrillation lead that can include a proximal end that can be connected to IMD 510 and a distal end that can be placed at a target location such as in the right ventricle (RV) 532 of the heart 501. The lead 508B can have a first pacing-sensing electrode 552 that can be located at distal end, a second pacing-sensing electrode 553 that can be located near the electrode 552, a first defibrillation coil electrode 554 that can be located near the electrode 553, and a second defibrillation coil electrode 555 that can be located at a distance from the distal end such as for superior vena cava (SVC) placement. The electrodes 552 through 555 can be electrically connected to the IMD 510 such as via separate conductors in the lead 508B. The electrodes 552 and 553 can allow for sensing of a ventricular electrogram and can optionally allow delivery of one or more ventricular pacing pulses, and electrodes 554 and 555 can allow for delivery of one or more ventricular cardioversion/defibrillation pulses. In an example, the lead 508B can include only three electrodes 552, 554 and 555. The electrodes 552 and 554 can be used for sensing or delivery of one or more ventricular pacing pulses, and the electrodes 554 and 555 can be used for delivery of one or more ventricular cardioversion or defibrillation pulses. The lead 508C can include a proximal end that can be connected to the IMD 510 and a distal end that can be configured to be placed at a target location such as in a left ventricle (LV) 534 of the heart 501. The lead 508C may be implanted through the coronary sinus 533 and may be placed in a coronary vein over the LV such as to allow for delivery of one or more pacing pulses to the LV. The lead 508C can include an electrode 561 that can be located at a distal end of the lead 508C and another electrode 562 that can be located near the electrode 561. The electrodes 561 and 562 can be electrically connected to the IMD 510 such as via separate conductors in the lead 508C such as to allow for sensing of the LV electrogram and optionally allow delivery of one or more resynchronization pacing pulses from the LV.

The IMD 510 can include an electronic circuit that can sense a physiologic signal. The physiologic signal can include an electrogram or a signal representing mechanical function of the heart 501. The hermetically sealed can 512 may function as an electrode such as for sensing or pulse delivery. For example, an electrode from one or more of the leads 508A - C may be used together with the can 512 such as for unipolar sensing of an electrogram or for delivering one or more pacing pulses. A defibrillation electrode from the lead 508B may be used together with the can 512 such as for delivering one or more cardioversion/defibrillation pulses. In an example, the IMD 510 can sense impedance such as between electrodes located on one or more of the leads 508A - C or the can 512. The IMD 510 can be configured to inject current between a pair of electrodes, sense the resultant voltage between the same or different pair of electrodes, and determine impedance using Ohm's Law. The impedance can be sensed in a bipolar configuration in which the same pair of electrodes can be used for injecting current and sensing voltage, a tripolar configuration in which the pair of electrodes for current injection and the pair of electrodes for voltage sensing can share a common electrode, or tetrapolar configuration in which the electrodes used for current injection can be distinct from the electrodes used for voltage sensing. In an example, the IMD 510 can be configured to inject current between an electrode on the RV lead 508B and the can 512, and to sense the resultant voltage between the same electrodes or between a different electrode on the RV lead 508B and the can 512. A physiologic signal can be sensed from one or more physiologic sensors that can be integrated within the IMD 510. The IMD 510 can also be configured to sense a physiologic signal from one or more external physiologic sensors or one or more external electrodes that can be coupled to the IMD 510. Examples of the physiologic signal can include one or more of heart rate, heart rate variability, intrathoracic impedance, intracardiac impedance, arterial pressure, pulmonary artery pressure, RV pressure, LV coronary pressure, coronary blood temperature, blood oxygen saturation, one or more heart sounds, physical activity or exertion level, physiologic response to activity, posture, respiration, body weight, or body temperature.

The IMD 510 can include a plethysmography sensor 565, such as a photoplethysmography sensor in the header 511 of the IMD 510. In other examples, the plethysmography sensor 565 can be coupled to the can 512, such as in or on a sidewall of the can 512, or in a window on the can 512, such that a photoplethysmography sensor can detect variations in light as blood volume changes in the subject.

The arrangement and functions of these leads and electrodes are described above by way of example and not by way of limitation. Depending on the need of the subject and the capability of the implantable device, other arrangements and uses of these leads and electrodes are anticipated and included herein.

The CRM system 500 can include a patient chronic condition-based HF assessment circuit, such as illustrated in the commonly assigned Qi An et al., U.S. Application Serial No. 14/55,392. The patient chronic condition-based HF assessment circuit can include a signal analyzer circuit and a risk stratification circuit. The signal analyzer circuit can receive patient chronic condition indicators and one or more physiologic signals from a patient and select one or more patient-specific sensor signals or signal metrics from the physiologic signals. The signal analyzer circuit can receive the physiologic signals from the patient using the electrodes on one or more of the leads 508A - C, or physiologic sensors deployed on or within the patient and communicated with the IMD 510. The risk stratification circuit can generate a composite risk index indicative of the probability of the patient later developing an event of worsening of HF (e.g., an HF decompensation event) such as using the selected patient-specific sensor signals or signal metrics. The HF decompensation event can include one or more early precursors of an HF decompensation episode, or an event indicative of HF progression such as recovery or worsening of HF status.

The external system 505 can allow for programming of the IMD 510 and can receives information about one or more signals acquired by IMD 510, such as can be received via a communication link 503. The external system 505 can include a local external IMD programmer. The external system 505 can include a remote patient management system that can monitor patient status or adjust one or more therapies such as from a remote location.

The communication link 503 can include one or more of an inductive telemetry link, a radio-frequency telemetry link, or a telecommunication link, such as an internet connection. The communication link 503 can provide for data transmission between the IMD 510 and the external system 505. The transmitted data can include, for example, real-time physiologic data acquired by the IMD 510, physiologic data acquired by and stored in the IMD 510, therapy history data or data indicating IMD operational status stored in the IMD 510, one or more programming instructions to the IMD 510 such as to configure the IMD 510 to perform one or more actions that can include physiologic data acquisition such as using programmably specifiable sensing electrodes and configuration, device self-diagnostic test, or delivery of one or more therapies.

The patient chronic condition-based HF assessment circuit, or other assessment circuit, may be implemented at the external system 505, which can be configured to perform HF risk stratification such as using data extracted from the IMD 510 or data stored in a memory within the external system 505. Portions of patient chronic condition-based HF or other assessment circuit may be distributed between the IMD 510 and the external system 505.

Portions of the IMD 510 or the external system 505 can be implemented using hardware, software, or any combination of hardware and software. Portions of the IMD 510 or the external system 505 may be implemented using an application-specific circuit that can be constructed or configured to perform one or more particular functions or can be implemented using a general-purpose circuit that can be programmed or otherwise configured to perform one or more particular functions. Such a general-purpose circuit can include a microprocessor or a portion thereof, a microcontroller or a portion thereof, or a programmable logic circuit, or a portion thereof. For example, a "comparator" can include, among other things, an electronic circuit comparator that can be constructed to perform the specific function of a comparison between two signals or the comparator can be implemented as a portion of a general-purpose circuit that can be driven by a code instructing a portion of the general-purpose circuit to perform a comparison between the two signals. While described with reference to the IMD 510, the CRM system 500 could include a subcutaneous medical device (e.g., subcutaneous ICD, subcutaneous diagnostic device), wearable medical devices (e.g., patch-based sensing device), or other external medical devices.

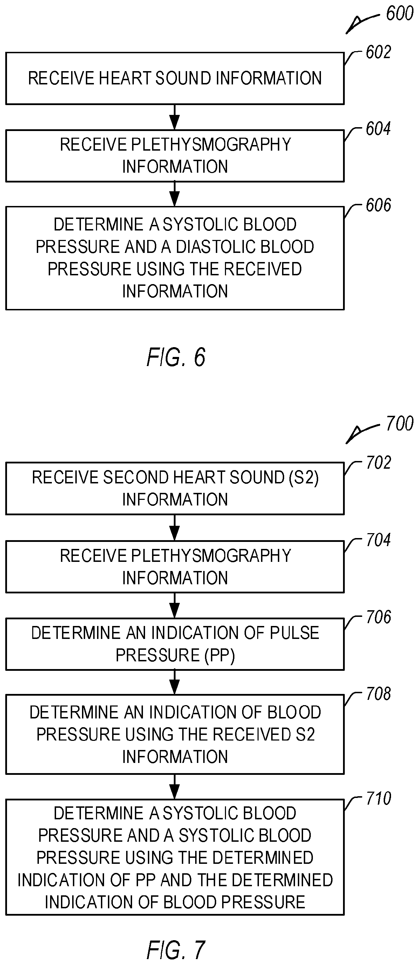

FIG. 6 illustrates a non-claimed example method 600 of determining a systolic blood pressure and a diastolic blood pressure of a subject using received physiologic information. At 602, heart sound information can be received, such as using a signal receiver circuit, from a heart sound sensor or one or more ambulatory devices or components of an external system. At 604, plethysmography information can be received, such as using the signal receiver circuit, from a plethysmography sensor (e.g., a photoplethysmography (PPG) sensor, etc.) or one or more ambulatory devices or components of an external system.

At 606, a systolic blood pressure of the subject and a diastolic blood pressure of the subject can be determined, such as by an assessment circuit, using the received heart sound and plethysmography information.

FIG. 7 illustrates a non-claimed example method 700 of determining a systolic blood pressure and a diastolic blood pressure of a subject using received physiologic information. At 702, second heart sound (S2) information can be received, such as using a signal receiver circuit, from a heart sound sensor or one or more ambulatory devices or components of an external system. At 704, plethysmography information can be received, such as using the signal receiver circuit, from a plethysmography sensor (e.g., a photoplethysmography (PPG) sensor, etc.) or one or more ambulatory devices or components of an external system.

At 706, an indication of a pulse pressure (PP) can be determined, such as by an assessment circuit, using the received plethysmography information. At 708, an indication of blood pressure (e.g., mean blood pressure) can be determined, such as by the assessment circuit, using the received S2 information.

At 710, the systolic blood pressure of the subject and the diastolic blood pressure of the subject can be determined, such as by the assessment circuit, using the determined indication of PP of the subject and the determined indication of blood pressure (e.g., mean blood pressure) of the subject.

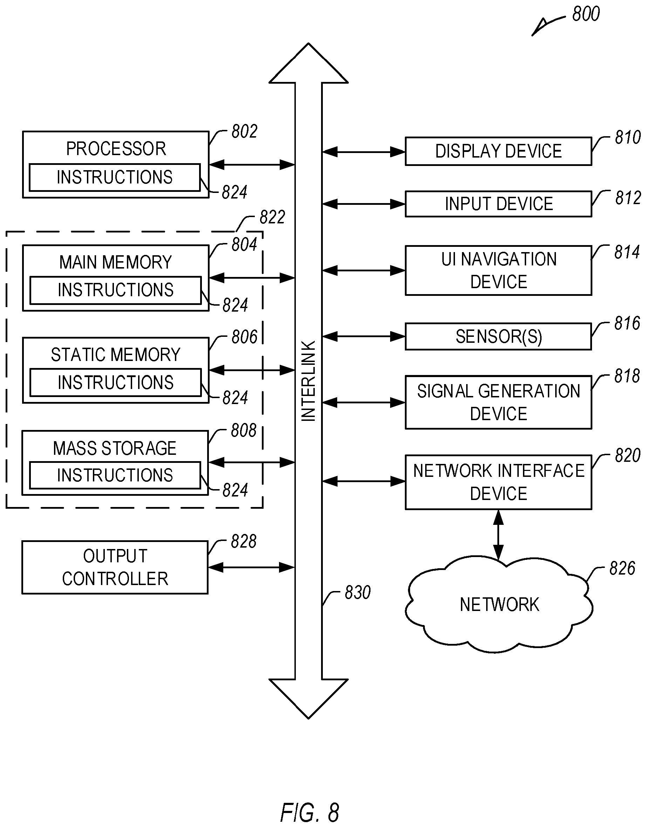

FIG. 8 illustrates a block diagram of an example machine 800 upon which any one or more of the techniques (e.g., methodologies) discussed herein may perform. Portions of this description may apply to the computing framework of one or more of the medical devices described herein, such as the IMD, the external programmer, etc.

Examples, as described herein, may include, or may operate by, logic or a number of components, or mechanisms in the machine 800. Circuitry (e.g., processing circuitry) is a collection of circuits implemented in tangible entities of the machine 800 that include hardware (e.g., simple circuits, gates, logic, etc.). Circuitry membership may be flexible over time. Circuitries include members that may, alone or in combination, perform specified operations when operating. In an example, hardware of the circuitry may be immutably designed to carry out a specific operation (e.g., hardwired). In an example, the hardware of the circuitry may include variably connected physical components (e.g., execution units, transistors, simple circuits, etc.) including a machine-readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuitry in hardware via the variable connections to carry out portions of the specific operation when in operation. Accordingly, in an example, the machine-readable medium elements are part of the circuitry or are communicatively coupled to the other components of the circuitry when the device is operating. In an example, any of the physical components may be used in more than one member of more than one circuitry. For example, under operation, execution units may be used in a first circuit of a first circuitry at one point in time and reused by a second circuit in the first circuitry, or by a third circuit in a second circuitry at a different time. Additional examples of these components with respect to the machine 800 follow.

In alternative embodiments, the machine 800 may operate as a standalone device or may be connected (e.g., networked) to other machines. In a networked deployment, the machine 800 may operate in the capacity of a server machine, a client machine, or both in server-client network environments. In an example, the machine 800 may act as a peer machine in peer-to-peer (P2P) (or other distributed) network environment. The machine 800 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

The machine (e.g., computer system) 800 may include a hardware processor 802 (e.g., a central processing unit (CPU), a graphics processing unit (GPU), a hardware processor core, or any combination thereof), a main memory 804, a static memory (e.g., memory or storage for firmware, microcode, a basic-input-output (BIOS), unified extensible firmware interface (UEFI), etc.) 806, and mass storage 808 (e.g., hard drive, tape drive, flash storage, or other block devices) some or all of which may communicate with each other via an interlink (e.g., bus) 830. The machine 800 may further include a display unit 810, an alphanumeric input device 812 (e.g., a keyboard), and a user interface (UI) navigation device 814 (e.g., a mouse). In an example, the display unit 810, input device 812, and UI navigation device 814 may be a touch screen display. The machine 800 may additionally include a signal generation device 818 (e.g., a speaker), a network interface device 820, and one or more sensors 816, such as a global positioning system (GPS) sensor, compass, accelerometer, or one or more other sensors. The machine 800 may include an output controller 828, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

Registers of the processor 802, the main memory 804, the static memory 806, or the mass storage 808 may be, or include, a machine-readable medium 822 on which is stored one or more sets of data structures or instructions 824 (e.g., software) embodying or utilized by any one or more of the techniques or functions described herein. The instructions 824 may also reside, completely or at least partially, within any of registers of the processor 802, the main memory 804, the static memory 806, or the mass storage 808 during execution thereof by the machine 800. In an example, one or any combination of the hardware processor 802, the main memory 804, the static memory 806, or the mass storage 808 may constitute the machine-readable medium 822. While the machine-readable medium 822 is illustrated as a single medium, the term "machine-readable medium" may include a single medium or multiple media (e.g., a centralized or distributed database, and/or associated caches and servers) configured to store the one or more instructions 824.

The term "machine-readable medium" may include any medium that is capable of storing, encoding, or carrying instructions for execution by the machine 800 and that cause the machine 800 to perform any one or more of the techniques of the present disclosure, or that is capable of storing, encoding, or carrying data structures used by or associated with such instructions. Nonlimiting machine-readable medium examples may include solid-state memories, optical media, magnetic media, and signals (e.g., radio frequency signals, other photon-based signals, sound signals, etc.). In an example, a non-transitory machine-readable medium comprises a machine-readable medium with a plurality of particles having invariant (e.g., rest) mass, and thus are compositions of matter. Accordingly, non-transitory machine-readable media are machine-readable media that do not include transitory propagating signals. Specific examples of non-transitory machine-readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 824 may be further transmitted or received over a communications network 826 using a transmission medium via the network interface device 820 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi®, IEEE 802.16 family of standards known as WiMax®), IEEE 802.15.4 family of standards, peer-to-peer (P2P) networks, among others. In an example, the network interface device 820 may include one or more physical jacks (e.g., Ethernet, coaxial, or phone jacks) or one or more antennas to connect to the communications network 826. In an example, the network interface device 820 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding, or carrying instructions for execution by the machine 800, and includes digital or analog communications signals or other intangible medium to facilitate communication of such software. A transmission medium is a machine-readable medium.

Various embodiments are illustrated in the figures above. One or more features from one or more of these embodiments may be combined to form other embodiments. Method examples described herein can be machine or computer-implemented at least in part. Some examples may include a computer-readable medium or machine-readable medium encoded with instructions operable to configure an electronic device or system to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times.

The above detailed description is intended to be illustrative, and not restrictive. The scope of the invention is determined by the appended claims.

**EP 3 820 359 B1**

**Claims**

1. A system (200; 300), comprising:

a signal receiver circuit (302) configured to receive second heart sound (S2) information of a subject and plethysmography information of the subject; and
an assessment circuit (304) configured to:

determine a mean blood pressure of the subject using the received S2 information;
determine an indication of pulse pressure of the subject using the received plethysmography information;
determine a systolic blood pressure of the subject as an increase to the determined mean blood pressure by a first function of the determined indication of pulse pressure of the subject;
determine a diastolic blood pressure of the subject as a decrease from the determined mean blood pressure by a second function of the determined indication of pulse pressure of the subject; and
determine the first function as a function of a rise time of the plethysmography signal and a time between the S2 heart sound and a peak time of the plethysmography signal.

2. The system (200; 300) of claim 1, wherein the assessment circuit (304) is configured to determine the first and second functions as different functions of the rise time of the plethysmography signal and the time between the S2 heart sound and a time at or near the peak of the plethysmography signal.

3. The system (200; 300) of claim 1 or 2, wherein the assessment circuit (304) is configured to determine the systolic blood pressure as $Systolic\ BP = BP_{S2} + a(PP)$ and determine the diastolic blood pressure as $Diastolic\ BP = BP_{S2} - (1-a)PP$,
wherein $BP_{S2}$ is the determined mean blood pressure of the subject, wherein PP is the determined indication of pulse pressure of the subject, wherein $a$ is $(T6-T5)/T6$, wherein $T6$ is the rise time of the plethysmography signal of the cardiac cycle, and wherein $T5$ is the time of the S2 heart sound of the cardiac cycle with respect to the time of the peak of the plethysmography signal of the cardiac cycle.

4. The system (200; 300) of any of the preceding claims, wherein the S2 information includes at least one of an S2 amplitude, energy, or time.

5. The system (200; 300) of any of the preceding claims, comprising:

a heart sound sensor (206) configured to detect heart sound information from the subject and to determine second heart sound (S2) information using the detected heart sound information; and
a plethysmography sensor (565) configured to detect plethysmography information from the subject,
wherein the signal receiver circuit (302) is configured to receive the determined second heart sound (S2) information from the heart sound sensor (206), and to receive the detected plethysmography information from the plethysmography sensor (565).

6. At least one machine-readable medium comprising instructions that, when performed by a medical device (202), cause the medical device (202) to perform operations comprising:

receiving second heart sound (S2) information of a subject and plethysmography information of the subject;
determining a mean blood pressure of the subject using the received S2 information;
determining an indication of pulse pressure of the subject using the received plethysmography information;
determining a systolic blood pressure of the subject as an increase to the determined mean blood pressure by a first function of the determined indication of pulse pressure of the subject;
determining a diastolic blood pressure of the subject as a decrease from the determined mean blood pressure by a second function of the determined indication of pulse pressure of the subject; and
determine the first function as a function of a rise time of the plethysmography signal and a time between the S2 heart sound and a peak time of the plethysmography signal.

7. The at least one machine-readable medium of claim 6, wherein the instructions, when performed by the medical device (202), cause the medical device (202) to perform operations comprising:
determining the first and second functions as different functions of the rise time of the plethysmography signal and a time between the S2 heart sound and a time at or near the peak of the plethysmography signal.

8. The at least one machine-readable medium of claim 6 or 7, wherein the instructions, when performed by the medical device (202), cause the medical device (202) to perform operations comprising:

determine the systolic blood pressure as *Systolic BP = BP$_{S2}$ + a(PP),*
determine the diastolic blood pressure *Diastolic BP = BP$_{S2}$ - (1-a)PP,*
wherein $BP_{S2}$ is the determined mean blood pressure of the subject, wherein PP is the determined indication of pulse pressure of the subject, wherein a is *(T6-T5)/T6,* wherein *T6* is the rise time of the plethysmography signal of the cardiac cycle, and wherein *T5* is the time of the S2 heart sound of the cardiac cycle with respect to the time of the peak of the plethysmography signal of the cardiac cycle.

**Patentansprüche**

1. System (200; 300), umfassend:

eine Signalempfängerschaltung (302), die eingerichtet ist, zweite Herzgeräuschinformationen (S2) einer Person und Plethysmographieinformationen der Person zu empfangen; und
eine Auswerteschaltung (304), die eingerichtet ist:

einen mittleren Blutdruck der Person unter Verwendung der empfangenen S2-Informationen zu bestimmen;
eine Pulsdruck-Indikation der Person unter Verwendung der empfangenen Plethysmographieinformationen zu bestimmen;
einen systolischen Blutdruck der Person als eine Erhöhung gegenüber dem bestimmten mittleren Blutdruck um eine erste Funktion der bestimmten Pulsdruck-Indikation der Person zu bestimmen;
einen diastolischen Blutdruck der Person als eine Abnahme gegenüber dem bestimmten mittleren Blutdruck um eine zweite Funktion der bestimmten Pulsdruck-Indikation der Person zu bestimmen; und
die erste Funktion als eine Funktion einer Anstiegszeit des Plethysmographiesignals und einer Zeit zwischen dem S2-Herzgeräusch und einer Spitzenzeit des Plethysmographiesignals zu bestimmen.

2. System (200; 300) nach Anspruch 1, wobei die Auswerteschaltung (304) eingerichtet ist, die erste und die zweite Funktion als unterschiedliche Funktionen der Anstiegszeit des Plethysmographiesignals und der Zeit zwischen dem S2-Herzgeräusch und einer Zeit bei oder nahe der Spitze des Plethysmographiesignals zu bestimmen.

3. System (200; 300) nach Anspruch 1 oder 2, wobei die Auswerteschaltung (304) eingerichtet ist, den systolischen Blutdruck als *Systolischer BP = BP$_{S2}$ + a(PP)* zu bestimmen und den diastolischen Blutdruck als *Diastolischer BP = BP$_{S2}$ -(1-a)PP* zu bestimmen,
wobei $BP_{S2}$ der bestimmte mittlere Blutdruck der Person ist, wobei *PP* die bestimmte Pulsdruck-Indikation der Person ist, wobei a *(T6- T5)/T6* ist, wobei *T6* die Anstiegszeit des Plethysmographiesignals des Herzzyklus ist, und wobei *T5* die Zeit des S2-Herzgeräuschs des Herzzyklus in Bezug auf die Zeit der Spitze des Plethysmographiesignals des Herzzyklus ist.

4. System (200; 300) nach einem der vorhergehenden Ansprüche, wobei die S2-Informationen zumindest eines von einer S2-Amplitude, Energie oder Zeit umfassen.

5. System (200; 300) nach einem der vorhergehenden Ansprüche, umfassend:

einen Herzgeräuschsensor (206), der eingerichtet ist, Herzgeräuschinformationen von der Person zu erfassen und zweite Herzgeräuschinformationen (S2) unter Verwendung der erfassten Herzgeräuschinformationen zu bestimmen; und
einen Plethysmographiesensor (565), der eingerichtet ist, Plethysmographieinformationen von der Person zu erfassen,
wobei die Signalempfängerschaltung (302) eingerichtet ist, die bestimmten zweiten Herzgeräuschinformationen (S2) von dem Herzgeräuschsensor (206) zu empfangen und die erfassten Plethysmographieinformationen von dem Plethysmographiesensor (565) zu empfangen.

6. Zumindest ein maschinenlesbares Medium, umfassend Anweisungen, die, bei Ausführung durch eine medizinische Einrichtung (202), die medizinische Einrichtung (202) veranlassen, Operationen durchzuführen, umfassend:

Empfangen von zweiten Herzgeräuschinformationen (S2) einer Person und von Plethysmographieinformationen der Person;

Bestimmen eines mittleren Blutdrucks der Person unter Verwendung der empfangenen S2-Informationen;

Bestimmen einer Pulsdruck-Indikation der Person unter Verwendung der empfangenen Plethysmographieinformationen;

Bestimmen eines systolischen Blutdrucks der Person als eine Erhöhung gegenüber dem bestimmten mittleren Blutdruck um eine erste Funktion der bestimmten Pulsdruck-Indikation der Person;

Bestimmen eines diastolischen Blutdrucks der Person als eine Abnahme gegenüber dem bestimmten mittleren Blutdruck um eine zweite Funktion der bestimmten Pulsdruck-Indikation der Person; und

die erste Funktion als eine Funktion einer Anstiegszeit des Plethysmographiesignals und einer Zeit zwischen dem S2-Herzgeräusch und einer Spitzenzeit des Plethysmographiesignals zu bestimmen.

7. Zumindest ein maschinenlesbares Medium nach Anspruch 6, wobei die Anweisungen, bei Durchführung durch die medizinische Einrichtung (202), die medizinische Einrichtung (202) veranlassen, Operationen durchzuführen, umfassend:

Bestimmen der ersten und zweiten Funktion als unterschiedliche Funktionen der Anstiegszeit des Plethysmographiesignals und einer Zeit zwischen dem S2-Herzgeräusch und einer Zeit bei oder nahe der Spitze des Plethysmographiesignals.

8. Zumindest ein maschinenlesbares Medium nach Anspruch 6 oder 7, umfassend Anweisungen, die bei Ausführung durch die medizinische Einrichtung (202), die medizinische Einrichtung (202) veranlassen, Operationen durchzuführen, umfassend:

Bestimmen des systolischen Blutdrucks als *Systolischer BP = $BP_{S2}$ + a(PP),*

Bestimmen des diastolischen Blutdrucks als *Diastolischer BP = $BP_{S2}$ -(1-a)PP,*

wobei $BP_{S2}$ der bestimmte mittlere Blutdruck der Person ist, wobei *PP* die bestimmte Pulsdruck-Indikation der Person ist, wobei a (T6-T5)/T6 ist, wobei T6 die Anstiegszeit des Plethysmographiesignals des Herzzyklus ist, und wobei T5 die Zeit des S2-Herzgeräusches des Herzzyklus in Bezug auf die Zeit der Spitze des Plethysmographiesignals des Herzzyklus ist.

## Revendications

1. Système (200 ; 300), comprenant :

un circuit de récepteur de signal (302) qui est configuré pour recevoir une seconde information de bruit du coeur (S2) d'un sujet et une information de pléthysmographie du sujet ; et

un circuit d'évaluation (304) qui est configuré pour :

déterminer une pression du sang moyenne du sujet en utilisant l'information S2 reçue ;

déterminer une indication de pression pulsée du sujet en utilisant l'information de pléthysmographie reçue ;

déterminer une pression du sang systolique du sujet en tant qu'augmentation jusqu'à la pression du sang moyenne déterminée au moyen d'une première fonction de l'indication de pression pulsée déterminée du sujet ;

déterminer une pression du sang diastolique du sujet en tant que diminution depuis la pression du sang moyenne déterminée au moyen d'une seconde fonction de l'indication de pression pulsée déterminée du sujet ; et

déterminer la première fonction en tant que fonction d'un temps de croissance du signal de pléthysmographie et d'un temps entre le bruit du coeur S2 et un temps de crête du signal de pléthysmographie.

2. Système (200 ; 300) selon la revendication 1, dans lequel le circuit d'évaluation (304) est configuré pour déterminer les première et seconde fonctions en tant que fonctions différentes du temps de croissance du signal de pléthysmographie et du temps entre l'information de bruit du coeur S2 et un temps au niveau ou à proximité de la crête du signal de pléthysmographie.

3. Système (200 ; 300) selon la revendication 1 ou 2, dans lequel le circuit d'évaluation (304) est configuré pour : déterminer la pression du sang systolique en tant que : *Systolic BP = $BP_{S2}$ + a(PP)* ; et déterminer la pression du sang diastolique en tant que : *Diastolic BP = $BP_{S2}$ - (1 - a)PP*;

dans lequel $BP_{S2}$ est la pression du sang moyenne déterminée du sujet, dans lequel $PP$ est l'indication de pression pulsée déterminée du sujet, dans lequel $a$ est *(T6 - TS)/T6,* où *T6* est le temps de croissance du signal de pléthysmographie du cycle cardiaque et où *T5* est le temps de l'information de bruit du coeur S2 du cycle cardiaque par rapport au temps de la crête du signal de pléthysmographie du cycle cardiaque.

4. Système (200 ; 300) selon l'une quelconque des revendications précédentes, dans lequel l'information S2 inclut au moins un élément pris parmi une amplitude S2, une énergie S2 et un temps S2.

5. Système (200 ; 300) selon l'une quelconque des revendications précédentes, comprenant :

un capteur de bruit du coeur (206) qui est configuré pour détecter l'information de bruit du coeur à partir du sujet et pour déterminer la seconde information de bruit du coeur (S2) en utilisant l'information de bruit du coeur détectée ; et
un capteur de pléthysmographie (565) qui est configuré pour détecter l'information de pléthysmographie à partir du sujet ;
dans lequel le circuit de récepteur de signal (302) est configuré pour recevoir la seconde information de bruit du coeur (S2) déterminée en provenance du capteur de bruit du coeur (206) et pour recevoir l'information de pléthysmographie détectée en provenance du capteur de pléthysmographie (565).

6. Au moins un support pouvant être lu par machine qui comprend des instructions qui, lorsqu'elles sont exécutées par un dispositif médical (202), ont pour effet que le dispositif médical (202) effectue les opérations comprenant :

la réception d'une seconde information de bruit du coeur (S2) d'un sujet et d'une information de pléthysmographie du sujet ;
la détermination d'une pression du sang moyenne du sujet en utilisant l'information S2 reçue ;
la détermination d'une indication de pression pulsée du sujet en utilisant l'information de pléthysmographie reçue ;
la détermination d'une pression du sang systolique du sujet en tant qu'augmentation jusqu'à la pression du sang moyenne déterminée au moyen d'une première fonction de l'indication de pression pulsée déterminée du sujet ;
la détermination d'une pression du sang diastolique du sujet en tant que diminution depuis la pression du sang moyenne déterminée au moyen d'une seconde fonction de l'indication de pression pulsée déterminée du sujet ; et
la détermination de la première fonction en tant que fonction d'un temps de croissance du signal de pléthysmographie et d'un temps entre le bruit du coeur S2 et un temps de crête du signal de pléthysmographie.

7. L'au moins un support pouvant être lu par machine selon la revendication 6, dans lequel les instructions, lorsqu'elles sont exécutées par le dispositif médical (202), ont pour effet que le dispositif médical (202) effectue les opérations comprenant :
la détermination des première et seconde fonctions en tant que fonctions différentes du temps de croissance du signal de pléthysmographie et d'un temps entre l'information de bruit du coeur S2 et un temps au niveau ou à proximité de la crête du signal de pléthysmographie.

8. L'au moins un support pouvant être lu par machine selon la revendication 6 ou 7, dans lequel les instructions, lorsqu'elles sont exécutées par le dispositif médical (202), ont pour effet que le dispositif médical (202) effectue les opérations comprenant :

la détermination de la pression du sang systolique en tant que : *Systolic BP = $BP_{S2}$ + a(PP)* ; et
la détermination de la pression du sang diastolique en tant que : *Diastolic BP = $BP_{S2}$ - (1 - a)PP ;*
dans lequel $BP_{S2}$ est la pression du sang moyenne déterminée du sujet, dans lequel $PP$ est l'indication de pression pulsée déterminée du sujet, dans lequel *a* est *(T6 - T5)/T6,* où *T6* est le temps de croissance du signal de pléthysmographie du cycle cardiaque et où *T5* est le temps de l'information de bruit du coeur S2 du cycle cardiaque par rapport au temps de la crête du signal de pléthysmographie du cycle cardiaque.

*FIG. 1*

FIG. 2

FIG. 3

400

EXTERNAL DEVICE 406
405
NETWORK 407
REMOTE DEVICE 408

401
410
411
402
411
411
403

FIG. 4

500

508A 508B 508C 565
510
511
512

555
533
531
552
551
532
554
553
552
501
562
561
534

503

EXTERNAL SYSTEM 505

FIG. 5

21

600

RECEIVE HEART SOUND INFORMATION — 602

↓

RECEIVE PLETHYSMOGRAPHY INFORMATION — 604

↓

DETERMINE A SYSTOLIC BLOOD PRESSURE AND A DIASTOLIC BLOOD PRESSURE USING THE RECEIVED INFORMATION — 606

*FIG. 6*

700

RECEIVE SECOND HEART SOUND (S2) INFORMATION — 702

↓

RECEIVE PLETHYSMOGRAPHY INFORMATION — 704

↓

DETERMINE AN INDICATION OF PULSE PRESSURE (PP) — 706

↓

DETERMINE AN INDICATION OF BLOOD PRESSURE USING THE RECEIVED S2 INFORMATION — 708

↓

DETERMINE A SYSTOLIC BLOOD PRESSURE AND A SYSTOLIC BLOOD PRESSURE USING THE DETERMINED INDICATION OF PP AND THE DETERMINED INDICATION OF BLOOD PRESSURE — 710

*FIG. 7*

FIG. 8

**EP 3 820 359 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62695511
- WO 2018099427 A1
- US 2017367659 A1
- US 7972275 B
- US 8048001 B
- US 55392 A, Qi An